(19) Europäisches Patentamt
European Patent Office
Office européen des brevets

(11) Numéro de publication: **0 055 192**
**B1**

(12) **FASCICULE DE BREVET EUROPEEN**

(45) Date de publication du fascicule du brevet: **08.08.84**

(51) Int. Cl.³: **C 07 C 53/12**, C 07 C 51/56

(21) Numéro de dépôt: **81420163.8**

(22) Date de dépôt: **30.10.81**

(54) **Procédé de préparation de l'anhydride acétique.**

(30) Priorité: **24.12.80 FR 8027942**

(43) Date de publication de la demande: **30.06.82 Bulletin 82/26**

(45) Mention de la délivrance du brevet: **08.08.84 Bulletin 84/32**

(84) Etats contractants désignés: **AT BE CH DE FR GB IT LI LU NL SE**

(56) Documents cités:
**GB - A - 2 007 666**
**US - A - 2 729 651**
**US - A - 4 002 678**
**US - A - 4 234 718**

(73) Titulaire: **RHONE-POULENC CHIMIE DE BASE, 25, quai Paul Doumer, F-92408 Courbevoie (FR)**

(72) Inventeur: **Gauthier-Lafaye, Jean, 21, rue Duguesclin, F-69006 Lyon (FR)**
Inventeur: **Perron, Robert, La Pécolière, F-69390 Charly (FR)**

(74) Mandataire: **Varniere-Grange, Monique et al, RHONE-POULENC RECHERCHES Service Brevets Chimie et Polymères Centre de Recherches de Saint-Fons B.P. 62, F-69192 St-Fons Cédex (FR)**

**EP 0 055 192 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de l'anhydride acétique par carbonylation de l'acétate de méthyle.

Il est bien connu que l'anhydride acétique peut être produit par carbonylation de l'acétate de méthyle, dans des conditions relativement sévères de pression en présence de complexes du nickel de formule

$[A_4M]_2NiX_4$

dans laquelle X représente un atome de brome ou d'iode, M un atome de phosphore ou d'azote, A étant, par exemple, un radical alkyle inférieur. (Of. brevet des ETATS UNIS D'AMERIQUE n° 2 729 651).

Ces complexes, obtenus par réaction d'halogénures de nickel sur des halogénures de phosphonium ou d'ammonium quaternaire, peuvent être engagés sous cette forme dans la réaction en cause, ou bien ils peuvent être formés in situ. Toutefois, l'efficacité de ce type de procédé est faible malgré les hautes pressions mises en oeuvre.

Plus récemment, il a été proposé des systèmes catalytiques permettant de carbonyler l'acétate de méthyle dans des conditions moins sévères de pression. Ainsi le brevet américain 4 002 678 décrit la carbonylation de l'acétate de méthyle en présence de nickel, de chrome, d'iode (ou d'un composé de l'iode) et d'une phosphine (ou d'une amine), sous une pression inférieure à 70 bars.

Parallèlement, il a été montré que le chrome n'est pas nécessaire dans un tel procédé lorsqu'on opère dans un acide carboxylique aliphatique comme solvant et sous réserve que l'iode (ou le composé de l'iode) soit engagé à la réaction dans une proportion telle que la fraction d'iode qui n'est chimiquement liée ni avec le nickel ni avec le promoteur (phosphine ou amine) soit d'au moins 0,2 mole (en iode élémentaire) à la fois par mole de composé du nickel et par mole de promoteur. (cf. la demande de brevet britannique n° 2 007 666).

Néanmoins, le développement à l'échelle industrielle de ces techniques récentes, dont l'intérêt de principe n'est pas contesté, semble compromis en raison de l'efficacité relativement faible des systèmes catalytiques en cause. Un obstacle supplémentaire au développement de ce type de procédés réside dans l'utilisation impérative de phosphines (ou d'amines) dont l'instabilité et le coût grèvent l'économie globale de ces procédés.

Il a maintenant été trouvé qu'il est possible, non seulement, d'augmenter l'efficacité de tels systèmes catalytiques à base de nickel, mais aussi qu'il n'est pas nécessaire, dans certaines conditions, de faire appel à des phosphines ou à des amines pour que les systèmes en cause développent une activité appréciable.

La présente invention a donc pour objet un procédé de préparation de l'anhydride acétique par carbonylation en phase liquide de l'acétate de méthyle dans un acide carboxylique en présence:

a) d'une quantité efficace de nickel,
b) d'iodure de méthyle,
c) d'un iodure ionique choisi dans le groupe constitué par les iodures d'ammonium quaternaire, les iodures de phosphonium quaternaire, l'iodure de sodium, l'iodure de potassium et l'iodure de césium, et
d) d'un cocatalyseur choisi parmi les sels du lithium et les sels des métaux alcalino-terreux.

Le procédé selon l'invention nécessite la présence d'une quantité efficace de nickel. N'importe quelle source de nickel peut être mise en oeuvre dans le cadre du présent procédé. On peut introduire le nickel sous sa forme métallique (nickel RANEY, par exemple) ou sous toute autre forme commode. A titre d'exemples de composés du nickel susceptibles d'être utilisés pour la mise en oeuvre du présent procédé, on peut citer: le carbonate, l'oxyde, l'hydroxyde, les halogénures, en particulier l'iodure, les carboxylates, en particulier l'acétate, de nickel. Conviennent également bien le nickel tétracarbonyle et le bis(triphénylphosphine) nickel dicarbonyle.

La quantité de nickel n'est pas critique. La teneur en nickel qui a une influence sur la vitesse de réaction est déterminée en fonction de la vitesse de réaction que l'on estime convenable, compte tenue des autres paramètres de la réaction. De manière générale, une quantité comprise entre 5 et 2000 milliatomes-grammes de nickel par litre de solution conduit à des résultats satisfaisants. Un opère de préférence avec une teneur comprise entre 20 et 1000 milliatomes-grammes de nickel par litre.

La mise en oeuvre de la présente invention nécessite également la présence d'iodure de méthyle dans le milieu réactionnel. Il n'est pas nécessaire de charger au départ ce composant du système catalytique et on peut faire appel, par exemple, à de l'iode libre, à de l'acide iodhydrique, à un iodure d'alkyle, différent de l'iodure de méthyle, ou à un iodure d'acyle. Comme les spécialistes le savent l'iode et ces types de composés iodés peuvent être considérés comme des précurseurs de l'iodure de méthyle dans la réaction en cause.

En général, l'iodure de méthyle est présent dans le milieu réactionnel à raison de 1 à 100 moles et, de préférence, à raison de 3 à 50 moles par atome-gramme de nickel se trouvant dans ledit milieu.

Le système catalytique mis en oeuvre dans le cadre du présent procédé comprend également un iodure ionique choisi dans le groupe constitué par les iodures d'ammonium quaternaire, les iodures de phosphonium quaternaire, l'iodure de sodium, l'iodure de potassium et l'iodure de césium.

La nature précise des iodures de phosphonium (ou d'ammonium) quaternaire n'est pas fondamentale et le choix parmi ces composés est davantage orienté par des considérations d'ordre pratique telles que la disponibilité, la commodité d'emploi et la solubilité dans le milieu réactionnel.

A ce titre, la Demanderesse préconise l'utilisation d'iodures d'ammonium ou de phosphonium quaternaire dont les cations sont représentés respectivement par les formules (I) et (II) ci-après:

$$R_1N^+(R_2)_3 \qquad (I)$$

$$R_1P^+(R_2)_3 \qquad (II)$$

dans lesquelles $R_1$ et $R_2$, identiques ou différents, représentent des radicaux alkyles linéaires ayant au maximum 4 atomes de carbone, $R_2$ pouvant en outre représenter un radical phényle, tolyle ou xylyle.

A titre d'exemples d'iodures d'ammonium quaternaire, convenant à la mise en oeuvre du présent procédé, on peut citer les iodures de tétraméthylammonium, triéthylméthylammonium, tributylméthylammonium, tributyl(n-propyl)ammonium, tétraéthylammonium et de tétrabutylammonium.

A titre d'exemples d'iodures de phosphonium quaternaire convenant à la mise en oeuvre du présent procédé, on peut citer les iodures de méthyltriphénylphosphonium, d'éthyltriphénylphosphonium, de méthyltrixylylphosphonium et de méthyltributylphosphonium.

Bien entendu ce type de composés, dont la présence est nécessaire à la mise en oeuvre de la présente invention, peut être formé in situ à partir de l'amine ou de la phosphine correspondante introduite, le cas échéant, sous la forme d'un complexe de nickel tel que le bis(triphénylphosphine)nickel dicarbonyle, et d'un iodure d'alkyle. Si l'on choisit ce mode opératoire, il conviendra de charger outre l'amine (ou la phosphine) en cause, la quantité d'iodure d'alkyle (le cas échéant, d'iodure de méthyle) nécessaire à sa quaternisation de telle sorte que cette transformation in situ ne se produise pas au détriment de l'iodure de méthyle dont la présence dans le milieu réactionnel est également nécessaire.

Les iodures de phosphonium quaternaire conviennent plus spécialement à la mise en oeuvre du présent procédé.

Comme indiqué ci-avant, l'iodure ionique peut être choisi également parmi les iodures de sodium, de potassium et de césium. Bien entendu, comme les spécialistes le comprendront aisément, il n'est pas nécessaire d'engager initialement les iodures de ces métaux, puisqu'ils peuvent se former in situ à partir d'iodure de méthyle (ou de ses précurseurs mentionnés en tête du présent mémoire) et par exemple d'un carboxylate du métal en cause. Il va de soi que cette transformation in situ ne devra pas non plus se faire au détriment de l'iodure de méthyle dont la présence dans le milieu réactionnel est nécessaire et que la remarque formulée à propos de la préparation in situ des iodures de phosphonium (ou d'ammonium) quaternaire s'applique aussi au cas présent.

Selon une variante avantageuse du présent procédé on fait appel aux iodures de sodium, de potassium ou de césium.

Si l'on désigne par $M_1$ le cation de l'iodure ionique, la quantité d'iodure ionique généralement présente dans le milieu réactionnel, est telle que l'on ait de 0,5 à 100 et, de préférence de 0,8 à 40 ions-grammes $M_1$ par atome-gramme de nickel également présent dans le milieu réactionnel. De manière avantageuse le rapport $M_1/Ni$ est compris entre 1 et 20.

Enfin, selon le présent procédé il est essentiel d'opérer en présence d'un cocatalyseur choisi parmi les sels de lithium et les sels des métaux alcalino-terreux.

Conviennent à la mise en oeuvre du présent procédé à la fois les sels minéraux et les sels organiques du lithium et des métaux alcalino-terreux. A titre d'exemple de sels utilisables dans le cadre de la présente invention on peut mentionner les hydroxydes, les carbonates, les iodures et les carboxylates renfermant au maximum 12 atomes de carbone, des métaux précités. Les sels de lithium et de magnésium s'avèrent plus particulièrement efficaces. Selon un aspect préféré du présent procédé on fait appel à un sel de lithium.

De manière générale les carboxylates des métaux précités, renfermant au maximum 12 atomes de carbone et, de préférence au maximum 4 atomes de carbone conviennent particulièrement bien à la mise en oeuvre de la présente invention. Les acétates, en particulier l'acétate de lithium, étant des cocatalyseurs particulièrement appropriés.

Si on désigne par $M_2$ le cation monovalent (ou divalent) de ces sels, cocatalyseurs dans le cadre du présent procédé, la quantité de cocatalyseur(s) généralement présente dans le milieu réactionnel est telle que l'on ait de 0,2 à 50 et, de préférence de 0,5 à 20 ions-grammes $M_2$ par atome-gramme de nickel.

Comme indiqué précédemment, le procédé, objet de la présente invention est conduit en phase liquide dans un acide carboxylique à titre de solvant.

La nature précise de l'acide carboxylique n'est pas fondamentale et le choix parmi les acides carboxyliques disponibles sera davantage orienté par des considérations économiques et/ou pratiques. A titre d'exemples d'acides carboxyliques utilisables comme solvants dans le cadre du présent procédé on peut citer l'acide acétique, l'acide propionique, l'acide butyrique et l'acide benzoïque.

En général, l'acide carboxylique représente de 10 à 90% et, de préférence de 20 à 80% du volume du liquide réactionnel.

Comme indiqué en tête du présent mémoire, la réaction est conduite en phase liquide sous une pression supérieure à la pression atmosphérique. En général, on opère sous une pression totale supérieure à 15 bars; il n'est pas utile d'atteindre 700 bars. Pour une bonne mise en oeuvre de l'invention, une pression totale de 25 à 200 bars est préconisée.

La température de réaction est généralement supérieure à 140° C, sans qu'il soit nécessaire d'atteindre 300° C. De bons résultats sont obtenus dans la gamme de températures allant de 160 à 220° C.

On met en oeuvre, de préférence, du monoxyde de carbone sous forme essentiellement pure, tel qu'il se présente dans le commerce. Toutefois, la présence d'impuretés telles que du dioxyde de carbone, de l'oxygène, du méthane et de l'azote peut être tolérée. La présence d'hydrogène n'est pas nuisible, même en des proportions relativement importantes.

En fin d'opération, l'anhydride acétique obtenu est séparé des autres constituants du milieu réactionnel par tout moyen approprié, par exemple par distillation.

Les exemples ci-après illustrent l'invention sans toutefois en limiter le domaine ou l'esprit.

Dans ce qui suit RR (%) désigne le nombre de moles d'anhydride acétique dosées pour 100 moles d'acétate de méthyle chargées.

Exemples 1 à 8

Une série d'essais, dont les conditions particulières ainsi que les résultats sont portés dans le tableau (I) ci-après, a été réalisée dans l'appareillage et selon le mode opératoire décrits plus particulièrement pour l'exemple 8.

Dans un autoclave en Hastelloy B2 et de 125 ml de capacité, on introduit:

- 25 ml d'acétate de méthyle
- 20 ml d'acide acétique
- 80 mMol d'iodure de méthyle
- 8 mAt-g de nickel sous forme d'acétate de nickel tétrahydraté
- 20 mMol d'iodure de méthyltriphénylphosphonium, qui sera désigné dans le tableau (I) ci-après par $MeP(Ph)_3I$, et
- 40 mMol d'acétate de lithium.

Après fermeture de l'autoclave, on établit une pression de 40 bars de monoxyde de carbone. L'agitation par un système de va-et-vient est mise en route et l'autoclave est porté, en 20 minutes environ, à 180° C, au moyen d'un four annulaire. La pression dans l'autoclave est alors de 64 bars; elle est ensuite maintenue constante et égale à 70 bars par des recharges successives de monoxyde de carbone.

Après 2 heures à 180° C, l'agitation et le chauffage sont arrêtés; l'autoclave est refroidi et dégazé. Le mélange réactionnel est alors dosé: il contient 19,58 g d'anhydride acétique. RR (%) = 61.

Tableau I

| N° | $P_T$ (bars) | Iodure Ionique Nature | mMol | Cocatalyseur nature | mMol | RR (%) |
|---|---|---|---|---|---|---|
| a | 70 | NaI | 20 | — | 0 | 6,5 |
| 1 | 70 | NaI | 20 | $Mg(OAc)_2$ | 20 | 11 |
| 2 | 70 | NaI | 20 | LiOAc | 40 | 13,5 |
| 3 | 70 | NaI | 53 | LiOAc | 40 | 38 |
| b | 70 | $MeP(Ph)_3I$ | 20 | — | 0 | 18 |
| 4 | 70 | $MeP(Ph)_3I$ | 20 | KOAc | 40 | 30 |
| 5 | 90 | $MeP(Ph)_3I$ | 20 | $Mg(OAc)_2$ | 40 | 38 |
| 6 | 70 | $MeP(Ph)_3I$ | 20 | LiOAc | 5 | 23 |
| 7 | 70 | $MeP(Ph)_3I$ | 20 | LiOAc | 10 | 33 |
| 8 | 70 | $MeP(Ph)_3I$ | 20 | LiOAc | 40 | 61 |

N. B.: Dans ce tableau $P_T$ est la pression totale et OAc désigne l'anion acétate.

Dans l'exemple 3, la productivité en anhydride acétique est de 120 grammes par heure et par litre (g/h × 1); en tenant compte de l'eau d'hydratation du catalyseur cette productivité (productivité potentielle) serait de 160 g/h × 1.

Dans l'exemple 8, la productivité est de 200 g/h × 1 et la »productivité potentielle« serait de 230 g/h × 1.

La comparaison des résultats obtenus dans les exemples 1 à 3, réalisés en présence d'un cocatalyseur, avec celui obtenu dans l'essai témoin (a), dans lequel on a omis le cocatalyseur, montre l'effet positif du cocatalyseur dans la réaction en cause.

La même conclusion s'impose lors de la comparaison entre les résultats des exemples 4 à 8 et celui obtenu dans l'essai témoin (b).

Exemple 9 et essais temoins (c) et (o)

Dans l'appareillage et selon le mode opératoire décrits ci-avant, une seconde série d'essais est réalisée sur une charge comprenant:

- 12,5 ml d'acétate de méthyle
- 11,25 ml d'acide acétique
- 19 mmol d'iodure de méthyle et
- 5 mAtg de nickel sous forme de nickel tétracarbonyle.

Les conditions particulières ainsi que les résultats obtenus à une température de 180°C sous une pression totale maintenue à 70 bars par des recharges de monoxyde de carbone sont portés dans le tableau II ci-après, dans lequel t désigne la durée de l'essai à la température précitée.

Tableau II

| N° | t en h | Iodure Ionique Nature | mMol | Cocatalyseur nature | mMol | RR (%) |
|---|---|---|---|---|---|---|
| c | 4 | NaI | 25 | — | 0 | 50 |
| d | 4 | — | 0 | LiI | 45 | 15 |
| 9 | 1 | NaI | 25 | LiI | 20 | 22 |

### Exemple 10

On reproduit l'exemple 2 ci-avant en remplaçant l'iodure de sodium par l'iodure de potassium, la pression totale étant de 90 bars.

RR (%) = 9,2
Productivité potentielle: 60 g/h × 1.

### Exemple 11

On reproduit l'exemple 2 ci-avant en remplaçant l'iodure de sodium par l'iodure de césium.

RR (%) = 4
Productivité potentielle: 45 g/h × 1.

### Exemple 12

On reproduit l'exemple 2 ci-avant en remplaçant l'acide acétique par 20 g d'acide benzoïque.

RR (%) = 33

### Exemple 13

On reproduit l'exemple 8 ci-avant en remplaçant l'acide acétique par 20 g d'acide benzoïque, la pression totale étant de 90 bars.

RR (%) = 70,5 (ce qui correspond à une productivité de 220 g/h × 1).

### Exemple 14

Dans l'autoclave et selon le mode opératoire décrits précédemment on réalise un essai sur une charge constituée de:

- 25 ml d'acétate de méthyle
- 20 ml d'acide acétique
- 80 mMol d'iodure de méthyle
- 8 mAtg de nickel sous forme de nickel tétracarbonyle
- 50 mMol d'iodure de sodium
- 50 mMol d'acétate de lithium.

Les résultats obtenus en 2 heures de réaction à 180° C, la pression totale étant maintenue à 60 bars par des recharges de monoxyde de carbone, sont les suivants:

RR (%) = 42 (ce qui correspond à une productivité de 130 g/h × 1).

## Revendications

1. Procédé de préparation de l'anhydride acétique par carbonylation en phase liquide de l'acétate de méthyle dans un acide carboxylique en présence:

a) d'une quantité efficace de nickel,
b) d'iodure de méthyle,
c) d'un iodure ionique choisi dans le groupe constitué par les iodures d'ammonium quaternaire, les iodures de phosphonium quaternaire, l'iodure de sodium, l'iodure de potassium et l'iodure de césium, et
d) d'un cocatalyseur choisi parmi les sels de lithium et les sels des métaux alcalino-terreux.

2. Procédé selon la revendication 1, caractérisé en ce que la pression totale est comprise entre 15 et 700 bars et, de préférence entre 25 et 200 bars.

3. Procédé selon la revendication 1 ou 2, caractérisé en ce que la température est comprise entre 140 et 300° C et, de préférence entre 160 et 220° C.

4. Procédé selon l'une quelconque des revendications précédentes, caractérisé en ce que la concentration du nickel est comprise entre 5 et 2000 milliatomes-gramme par litre de milieu réactionnel.

5. Procédé selon la revendication 4, caractérisé en ce que la concentration du nickel est comprise entre 20 et 1000 milliatomes-gramme par litre de milieu réactionnel.

6. Procédé selon l'une quelconque des revendications précédentes, caractérisé en ce que l'iodure de méthyle est présent dans le milieu réactionnel à raison de I à 100 moles par atome-gramme de nickel.

7. Procédé selon la revendication 6, caractérisé en ce que l'iodure de méthyle est présent dans le milieu réactionnel à raison de 3 à 50 moles par atome-gramme de nickel.

8. Procédé selon l'une quelconque des revendications précédentes, caractérisé en ce que le rapport molaire $M_1/Ni$ ($M_1$ représentant le cation de l'iodure ionique) est compris entre 0,5 et 100 et, de préférence entre 0,8 et 40.

9. Procédé selon l'une quelconque des revendications précédentes, caractérisé en ce que l'iodure ionique est un iodure de phosphonium quaternaire.

10. Procédé selon l'une quelconque des revendications 1 à 8, caractérisé en ce que l'iodure ionique est choisi parmi l'iodure de sodium, l'iodure de potassium et l'iodure de césium.

11. Procédé selon l'une quelconque des revendications précédentes, caractérisé en ce que le cocatalyseur est un carboxylate.

12. Procédé selon l'une quelconque des revendications précédentes, caractérisé en ce que le cocatalyseur est un acétate.

13. Procédé selon l'une quelconque des revendications précédentes, caractérisé en ce que le cocatalyseur est un sel de lithium.

14. Procédé selon l'une quelconque des revendications précédentes, caractérisé en ce que le rapport molaire $M_2/Ni$ ($M_2$ représentant le cation du cocatalyseur) est compris entre 0,2 et 50 et, de préférence entre 0,5 et 20.

15. Procédé selon l'une quelconque des revendications précédentes, caractérisé en ce que l'acide carboxylique est l'acide acétique.

16. Procédé selon l'une quelconque des revendications précédentes, caractérisé en ce que l'acide carboxylique est l'acide benzoïque.

17. Procédé selon l'une quelconque des revendications précédentes, caractérisé en ce que l'acide carboxylique représente de 10 à 90% et, de préférence de 20 à 80% du volume du liquide réactionnel.

**Patentansprüche**

1. Verfahren zur Herstellung von Essigsäureanhydrid durch Carbonylieren von Methylacetat in flüssiger Phase in einer Carbonsäure in Gegenwart von:

a) einer wirksamen Menge Nickel,
b) Methyliodid,
c) einem ionischen Iodid, ausgewählt aus der Gruppe, bestehend aus den quaternären Ammoniumiodiden, quaternären Phosphoniumiodiden, Natriumiodid, Kaliumiodid und Caesiumiodid und
d) einem Cokatalysator, ausgewählt unter den Lithiumsalzen und Erdalkalisalzen.

2. Verfahren nach Anspruch 1, dadurch gekennzeichnet, daß der Gesamtdruck 15 bis 700 bar, vorzugsweise 25 bis 200 bar beträgt.

3. Verfahren nach Anspruch 1 oder 2, dadurch gekennzeichnet, daß die Temperatur 140 bis 300° C, vorzugsweise 160 bis 220° C beträgt.

4. Verfahren nach einem der vorangehenden Ansprüche, dadurch gekennzeichnet, daß die Nickelkonzentration 5 bis 2000 mgAtom je Liter Reaktionsmedium ausmacht.

5. Verfahren nach Anspruch 4, dadurch gekennzeichnet, daß die Nickelkonzentration 20 bis 1000 mgAtom je Liter Reaktionsmedium ausmacht.

6. Verfahren nach einem der vorangehenden Ansprüche, dadurch gekennzeichnet, daß das Methyliodid im Reaktionsmedium in einer Menge von 1 bis 100 mol/gAtom Nickel vorhanden ist.

7. Verfahren nach Anspruch 6, dadurch gekennzeichnet, daß das Methyliodid im Reaktionsmedium in einer Menge von 3 bis 50 mol/gAtom Nickel vorhanden ist.

8. Verfahren nach einem der vorangehenden Ansprüche, dadurch gekennzeichnet, daß das Molverhältnis $M_1/Ni$ ($M_1$ bedeutet das Kation des ionischen Iodids) 0,5 bis 100, vorzugsweise 0,8 bis 40 beträgt.

9. Verfahren nach einem der vorangehenden Ansprüche, dadurch gekennzeichnet, daß das ionische Iodid ein quaternäres Phosphoniumiodid ist.

10. Verfahren nach einem der Ansprüche 1 bis 8, dadurch gekennzeichnet, daß das ionische Iodid unter Natriumiodid, Kaliumiodid und Caesiumiodid ausgewählt wird.

11. Verfahren nach einem der vorangehenden Ansprüche, dadurch gekennzeichnet, daß der Cokatalysator ein Carboxylat ist.

12. Verfahren nach einem der vorangehenden Ansprüche, dadurch gekennzeichnet, daß der Cokatalysator ein Acetat ist.

13. Verfahren nach einem der vorangehenden Ansprüche, dadurch gekennzeichnet, daß der Coka-

talysator ein Lithiumsalz ist.

14. Verfahren nach einem der vorangehenden Ansprüche, dadurch gekennzeichnet, daß das Molverhältnis $M_2$/Ni ($M_2$ bedeutet das Kation des Cokatalysators) 0,2 bis 50, vorzugsweise 0,5 bis 20 beträgt.

15. Verfahren nach einem der vorangehenden Ansprüche, dadurch gekennzeichnet, daß die Carbonsäure die Essigsäure ist.

16. Verfahren nach einem der vorangehenden Ansprüche, dadurch gekennzeichnet, daß die Carbonsäure die Benzoesäure ist.

17. Verfahren nach einem der vorangehenden Ansprüche, dadurch gekennzeichnet, daß die Carbonsäure 10 bis 90%, vorzugsweise 20 bis 80% des Volumens der Reaktionsflüssigkeit ausmacht.

**Claims**

1. Process for the preparation of acetic anhydride by the carbonylation of methyl acetate in the liquid phase, in a carboxylic acid, in the presence of:

a) an effective amount of nickel,
b) methyl iodide,
c) an ionic iodide chosen from the group comprising quaternary ammonium iodides, quaternary phosphonium iodides, sodium iodide, potassium iodide and caesium iodide, and
d) a co-catalyst chosen from amongst lithium salts and alkaline earth metal salts.

2. Process according to Claim 1, characterised in that the total pressure is between 15 and 700 bars and preferably between 25 and 200 bars.

3. Process according to Claim 1 or 2, characterised in that the temperature is between 140 and 300° C and preferably between 160 and 220° C.

4. Process according to any one of the preceding claims, characterised in that the concentration of the nickel is between 5 and 2,000 milligram atoms per litre of reaction medium.

5. Process according to Claim 4, characterised in that the concentration of the nickel is between 20 and 1,000 milligram atoms per litre of reaction medium.

6. Process according to any one of the preceding claims, characterised in that the methyl iodide is present in the reaction medium in an amount of 1 to 100 mols per gram atom of nickel.

7. Process according to Claim 6, characterised in that the methyl iodide is present in the reaction medium in an amount of 3 to 50 mols per gram atom of nickel.

8. Process according to any one of the preceding claims, characterised in that the molar ratio $M_1$/Ni ($M_1$ representing the cation of the ionic iodide) is between 0.5 and 100 and preferably between 0.8 and 40.

9. Process according to any one of the preceding claims, characterised in that the ionic iodide is a quaternary phosphonium iodide.

10. Process according to any one of Claims 1 to 8, characterised in that the ionic iodide is chosen from amongst sodium iodide, potassium iodide and caesium iodide.

11. Process according to any one of the preceding claims, characterised in that the co-catalyst is a carboxylate.

12. Process according to any one of the preceding claims, characterised in that the co-catalyst is an acetate.

13. Process according to any one of the preceding claims, characterised in that the co-catalyst is a lithium salt.

14. Process according to any one of the preceding claims, characterised in that the molar ratio $M_2$/Ni ($M_2$ representing the cation of the co-catalyst) is between 0.2 and 50 and preferably between 0.5 and 20.

15. Process according to any one of the preceding claims, characterised in that the carboxylic acid is acetic acid.

16. Process according to any one of the preceding claims, caracterised in that the carboxylic acid is benzoic acid.

17. Process according to any one of the preceding claims, characterised in that the carboxylic acid represents from 10 to 90% and preferably from 20 to 80% of the volume of the reaction liquid.